# EUROPÄISCHE PATENTSCHRIFT

(11) **EP 1 773 825 B1**
(45) Veröffentlichungstag und Bekanntmachung des Hinweises auf die Patenterteilung: **23.01.2008**
(21) Anmeldenummer: 05761599.9
(22) Anmeldetag: 13.07.2005
(51) Int. Cl.: C07D 413/14, A01N 47/38

(54) **DIOXAZINYL-SUBSTITUIERTE THIENYLSULFONYLAMINOCARBONYLVERBINDUNGEN**
DIOXAZINYL-SUBSTITUTED THIENYLSULPHONYLAMINOCARBONYL COMPOUNDS
COMPOSES THIENYLSULFONYLAMINOCARBONYLES SUBSTITUES PAR DIOXAZINYLE

(30) Priorität: 28.07.2004 DE 102004036551
(43) Veröffentlichungstag der Anmeldung: 18.04.2007
(73) Patentinhaber: Bayer CropScience Aktiengesellschaft, 40789 Monheim (DE)
(72) Erfinder: GESING, Ernst, R., F., 40699 Erkrath-Hochdahl (DE); FEUCHT, Dieter, 65760 Eschborn (DE); KEHNE, Heinz, 65719 Hofheim (DE); AULER, Thomas, 42799 Leichlingen (DE); HILLS, Martin, 65510 Idstein (DE)
(74) Vertreter: Schwenk, Norbert
(86) Internationale Anmeldenummer: PCT/EP2005/007580
(87) Internationale Veröffentlichungsnummer: WO 2006/012982

(56) Entgegenhaltungen:
- EP-A- 0 301 784
- EP-A- 1 561 378
- WO-A-03/037086
- WO-A-03/056922
- WO-A1-98/07721
- US-A- 5 476 936

## Beschreibung

Die Erfindung betrifft neue substituierte Thienylsulfonylaminocarbonylverbindungen, Verfahren und Zwischenprodukte zu ihrer Herstellung und ihre Verwendung als Herbizide.

Es ist bereits bekannt, dass bestimmte Dioxazinyl-substituierte Thienylsulfonylaminocarbonylverbindungen herbizide Eigenschaften aufweisen (vgl. US 5,476,936). Die herbizide Wirksamkeit dieser bekannten Verbindungen ist jedoch nicht in allen Belangen zufriedenstellend.

Es wurden nun die neuen substituierten Thienylsufonylaminocarbonylverbindungen der allgemeinen Formel (I) 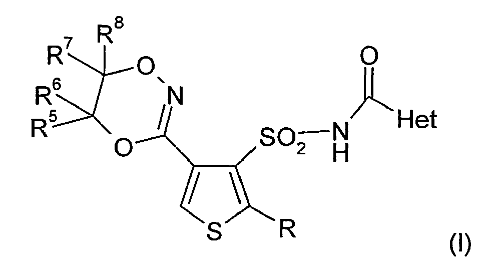 in welcher
- Het: für 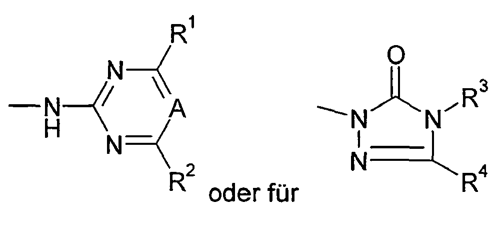 steht,
- A: für Stickstoff oder eine CH-Gruppierung steht,
- R: für Wasserstoff, Cyano, Nitro, Halogen, für jeweils gegebenenfalls durch Cyano, Halogen oder C₁-C₄-Alkoxy substituiertes Alkyl, Alkoxy, Alkoxycarbonyl, Alkylthio, Alkylsulfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
- R¹: für Wasserstoff, für Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
- R²: für Wasserstoff, für Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
- R³: für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Brom, Cyano, C₁-C₄-Alkoxy oder C₁-C₄-Alkoxy-carbonyl substituiertes Alkoxy, Alkylamino oder Alkylcarbonylamino mit jeweils 1 bis 6 Kohlenstoffatomen in der Alkylgruppe, für Alkenyloxy mit 3 bis 6 Kohlenstoffatomen, für Dialkylamino mit jeweils 1 bis 4 Kohlenstoffatomen in den Alkylgruppen, für jeweils gegebenenfalls durch Fluor, Chlor, Brom, Cyano und/oder C₁-C₄-Alkyl substituiertes Cycloalkyl, Cycloalkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl und/oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht,
- R⁴: für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Cyano, C₁-C₄-Alkoxy oder C₁-C₄-Alkoxy-carbonyl substituiertes Alkoxy, Alkylthio, Alkylamino oder Alkylcarbonylamino mit jeweils 1 bis 6 Kohlenstoffatomen in der Alkylgruppe, für Alkenyloxy, Alkinyloxy, Alkenylthio, Alkinylthio, Alkenylamino oder Alkinylamino mit jeweils 3 bis 6 Kohlenstoffatomen in der Alkenyl- oder Alkinylgruppe, für Dialkylamino mit jeweils 1 bis 4 Kohlenstoffatomen in den Alkylgruppen, für jeweils gegebenenfalls durch Fluor, Chlor, Brom, Cyano und/oder C₁-C₄-Alkyl substituiertes Cycloalkyl, Cycloalkenyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in der Cycloalkyl- bzw. Cycloalkenylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil steht, und
- R⁵, R⁶, R⁷ und R⁸: unabhängig voneinander für Wasserstoff, Halogen, Cyano oder Thiocyanato oder für gegebenenfalls durch Halogen substituiertes Alkyl, Alkoxy, Alkylthio, Alkylsulfinyl, Alkylsulfonyl Alkylamino, Alkylcarbonyl, Alkoxycarbonyl oder Alkylaminocarbonyl mit jeweils 1 bis 3 Kohlenstoffatomen im Alkylteil stehen,
sowie Salze von Verbindungen der Formel (I) gefunden.

Gesättigte oder ungesättigte Kohlenwasserstoffgruppierungen, wie Alkyl, Alkenyl oder Alkinyl, sind - auch in Verknüpfungen mit Heteroatomen, wie in Alkoxy - soweit möglich jeweils geradkettig oder verzweigt.

Gegebenenfalls substituierte Reste können einfach oder mehrfach substituiert sein, wobei bei Mehrfachsubstitution die Substituenten gleich oder verschieden sein können.
- R: steht bevorzugt für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, Methoxycarbonyl, Ethoxycarbonyl, n- oder i-Propoxycarbonyl, , Methylthio, Ethylthio, n- oder i-Propylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy.
- R: steht besonders bevorzugt für Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy.
- R: steht am meisten bevorzugt für Methyl, Ethyl, n- oder i-Propyl.
- R¹: steht bevorzugt für Wasserstoff, Fluor, Chlor, Brom, Iod, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino.
- R¹: steht besonders bevorzugt für Fluor, Chlor, Brom, oder Iod, oder für gegebenenfalls durch Fluor oder Chlor substituiertes Methyl, Ethyl, n- oder i-Propyl, Methoxy, Ethoxy, n- oder i-Propoxy oder für Dimethylamino.
- R²: steht bevorzugt für Fluor, Chlor, Brom, Iod oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino.
- R²: steht besonders bevorzugt für Fluor, Chlor, Brom, oder Iod, oder für gegebenenfalls durch Fluor oder Chlor substituiertes Methyl, Ethyl, n- oder i-Propyl, Methoxy, Ethoxy, n- oder i-Propoxy oder für Dimethylamino.
- R³: steht bevorzugt für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Di-ethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl.
- R³: steht besonders bevorzugt für Methyl, Ethyl, n- oder i-Propyl oder Cyclopropyl.
- R⁴: steht bevorzugt für Wasserstoff, Hydroxy, Mercapto, Amino, Cyano, Fluor, Chlor, Brom, für jeweils gegebenenfalls durch Fluor, Chlor, Cyano, Methoxy, Ethoxy, n- oder i-Propoxy, Acetyl, Propionyl, n- oder i-Buyroyl, Methoxycarbonyl, Ethoxycarbonyl, n- oder i-Propoxy-carbonyl substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Ethinyl, Propinyl oder Butinyl, für jeweils gegebenenfalls durch Fluor, Chlor, Cyano, Methoxy, Ethoxy, n- oder i-Propoxy, Methoxycarbonyl, Ethoxy-carbonyl, n- oder i-Propoxy-carbonyl substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Acetylamino oder Propionylamino, für Propenyloxy, Butenyloxy, Ethinyloxy, Propinyloxy, Butinyloxy, Propenylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Brom, Methyl, Trifluormethyl, Methoxy oder Methoxy-carbonyl substituiertes Phenyl, Benzyl, Phenoxy, Benzyloxy, Phenylthio, Benzylthio, Phenylamino oder Benzylamino.
- R⁴: steht besonders bevorzugt für Methoxy, Ethoxy, n- oder i-Propoxy, n-, i-, s-oder t-Butoxy.
- R⁵, R⁶, R⁷ und R⁸: stehen bevorzugt unabhängig voneinander für Wasserstoff oder Methyl.
- R⁵, R⁶, R⁷ und R⁸: stehen besonders bevorzugt für Wasserstoff.

Gegenstand der Erfindung sind vorzugsweise auch die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (1), in welcher A, Het, R, R¹, R², R³, R⁴, R⁵, R⁶, R⁷ und R⁸ die oben vorzugsweise angegebene Bedeutung haben.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Erfindungsgemäß bevorzugt sind diejenigen Verbindungen der Formel (I), bei welchen eine Kombination der vorstehend als bevorzugt aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Die neuen substituierten Thienylsulfonylaminocarbonylverbindungen der allgemeinen Formel (I) zeichnen sich durch starke herbizide Wirksamkeit aus.

Man erhält die neuen substituierten Thienylsulfonylaminocarbonylverbindungen der allgemeinen Formel (I), wenn man
(a) substituierte Aminoazine der allgemeinen Formel (II) 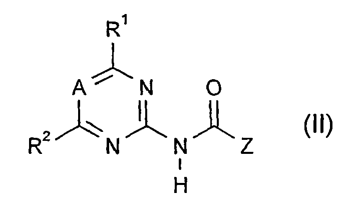 in welcher
   A, R¹ und R² die oben angegebene Bedeutung haben, und
   Z für Halogen, Alkoxy oder Aryloxy steht,
   mit Thiophenderivaten der allgemeinen Formel (III) 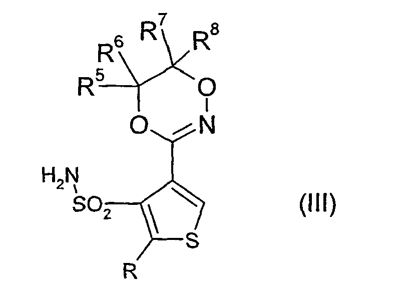 in welcher
   R und R⁵ bis R⁸ die oben angegebene Bedeutung haben,
   gegebenenfalls in Gegenwart eines Reaktionshilfsmittels und gegebenenfalls in Gegenwart eines Verdünnungsmittels umsetzt,
   oder (b) substituierte Thiophen-3-sulfonamide der allgemeinen Formel (III) 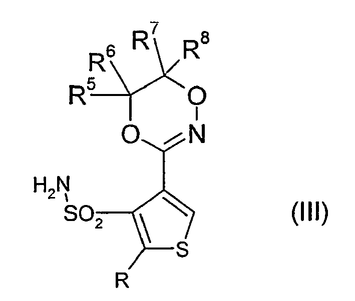 in welcher R und R⁵ bis R⁸ die oben angegebene Bedeutung haben,
   mit substituierten Triazolinonen der allgemeinen Formel (IV) 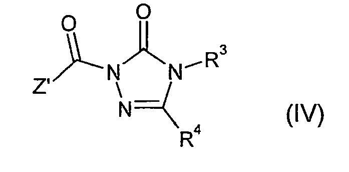 in welcher
   - R³ und R⁴: die oben angegebene Bedeutung haben und
   - Z': für Halogen, Alkoxy, Aryloxy oder Arylalkoxy steht,
   gegebenenfalls in Gegenwart eines Reaktionshilfsmittels und gegebenenfalls in Gegenwart eines Verdünnungsmittels umsetzt,
   und gegebenenfalls die nach den Verfahren (a) oder (b) erhaltenen Verbindungen der Formel (I) nach üblichen Methoden in Salze überführt.

Verwendet man beispielsweise 2-Methoxycarbonylamino-4-methoxy-6-trifluormethyl-1,3,5-triazin und 2-Ethyl-4-(5,6-dihydro-[1,4,2]-dioxazin-3-yl)-thiophen-3-sulfonamid als Ausgangsstoffe, so kann der Reaktionsablauf beim erfindungsgemäßen Verfahren (a) durch das folgende Formelschema skizziert werden:

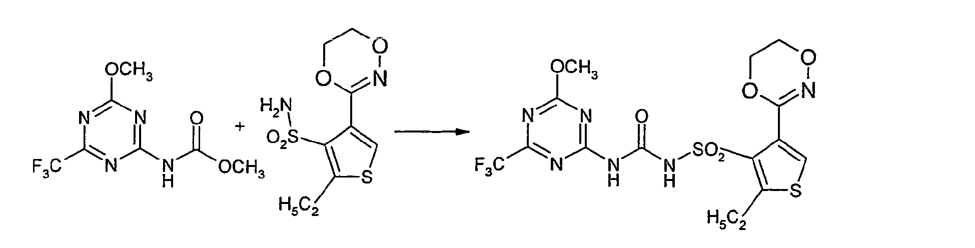

Die beim erfindungsgemäßen Verfahren zur Herstellung der Verbindungen der Formel (I) als Ausgangsstoffe zu verwendenden substituierten Aminoazine sind durch die Formel (II) allgemein definiert. In der Formel (II) haben A, R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, R¹ und R² angegeben wurden; Z steht vorzugsweise für Fluor, Chlor, Brom, C₁-C₄-Alkoxy oder Phenoxy, insbesondere für Chlor, Methoxy, Ethoxy oder Phenoxy.

Die Ausgangsstoffe der Formel (II) sind bekannt und/oder können nach an sich bekannten Verfahren hergestellt werden (vgl. US 4,690,707, DE 19501174).

Die beim erfindungsgemäßen Verfahren weiter als Ausgangsstoffe zu verwendenden Thiophenderivate sind durch die Formel (III) allgemein definiert. In der Formel (III) haben R und R⁵ bis R⁸ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R und R⁵ bis R⁸ angegeben wurden.

Die substituierten Thiophen-3-sulfonamide der allgemeinen Formel (III) sind noch nicht aus der Literatur bekannt, sie sind als solche ebenfalls Gegenstand der vorliegenden Erfindung. Unter den Verbindungen der Formel (III) sind solche bevorzugt, bei denen R nicht für Wasserstoff steht.

Man erhält die substituierten Thiophen-3-sulfonamide der allgemeinen Formel (III), wenn man substituierte Thiophen-3-sulfonamide der allgemeinen Formel (V) 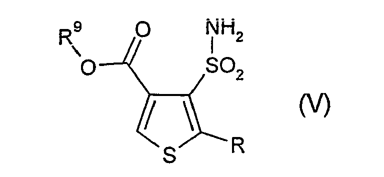 in welcher
R die oben angegebene Bedeutung hat und R⁹ für C₁-C₄-Alkyl steht,
mit Hydroxylaminhydrochlorid und einem substituierten Alkan der Formel (VI) 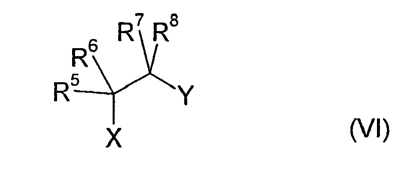 worin R⁵ bis R⁸ die oben angegebene Bedeutung haben und
X und Y unabhängig voneinander für Halogen, C₁-C₆-Alkylcarbonyloxy, C₆-C₁₂-Arylcarbonyloxy C₁-C₆-Alkylsulfonyloxy oder C₁-C₆-Arylsulfonyloxy stehen,
gegebenenfalls in Gegenwart eines Verdünnungsmittels und gegebenenfalls in Gegenwart eines Reaktionshilfsmittels umsetzt (vgl. das Herstellungsbeispiel). Analoge Verfahren sind auch aus der US 5,476,936 (vgl. Spalten 11/12) bekannt.

Die Ausgangsstoffe der Formel (V) sind bekannt und/oder können nach an sich bekannten Verfahren hergestellt werden (vgl. WO 01/05788, WO 01/10863).

Die Alkane der Formel (VI) sind als Synthesechemikalien kommerziell erhältlich oder können nach an sich bekannten Verfahren hergestellt werden (vgl. US 5,476,936, Spalte 13).

Verwendet man beispielsweise 2-Methyl-4-(5,6-dihydro-[1,4,2]-dioxazin-3-yl)-thiophen-3-sulfonamid und 4,5-Dimethoxy-2-phenoxycarbonyl-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

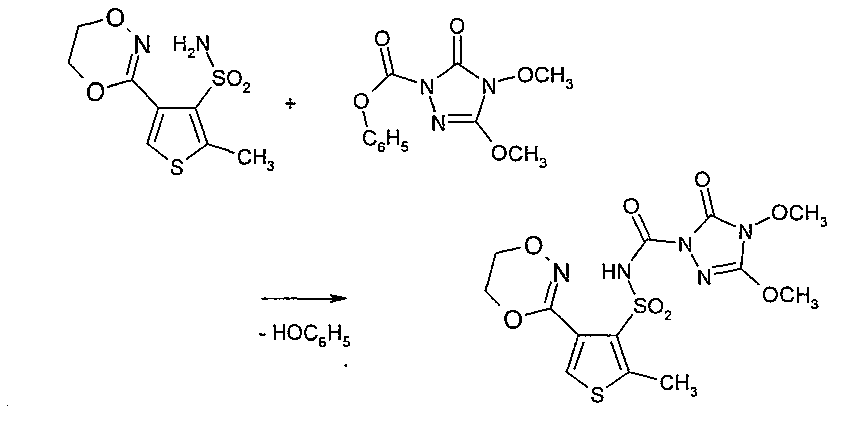

Die beim erfindungsgemäßen Verfahren (b) zur Herstellung von Verbindungen der allgemeinen Formel (I) als Ausgangsstoffe zu verwendenden substituierten Thiophen-3-sulfonamide sind durch die Formel (III) allgemein definiert. Es handelt sich um dieselben Verbindungen der Formel (III) die gemäß des erfindungsgemäßen Verfahrens (a) verwendet werden.

Die beim erfindungsgemäßen Verfahren (b) zur Herstellung von Verbindungen der allgemeinen Formel (I) weiter als Ausgangsstoffe zu verwendenden substituierten Triazolinone sind durch die Formel (IV) allgemein definiert. In der allgemeinen Formel (IV) haben R³ und R⁴ vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für R³ und R³ angegeben worden sind.

Die Ausgangsstoffe der allgemeinen Formel (IV) sind bekannt und/oder können nach an sich bekannten Verfahren hergestellt werden (vgl. auch Hinweise in WO 01/05788). Z' in der Formel (IV) steht bevorzugt für Chlor, Brom, Methoxy, Ethoxy, Phenoxy oder Benzyloxy.

Ais Verdünnungsmittel zur Durchführung der erfindungsgemäßen Verfahren (a) und (b) und des Verfahrens zur Herstellung derZwischenprodukte der Formel (III) kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester, sowie Sulfoxide, wie Dimethylsulfoxid.

Die erfindungsgemäßen Verfahren (a) und (b) sowie das Verfahren zur Herstellung der Zwischenprodukte der Formel (III) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetall- -hydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im Allgemeinen arbeitet man bei Temperaturen zwischen -20°C und +150°C, vorzugsweise zwischen -10°C und +120°C.

Die Reaktionstemperaturen können bei der Durchführung des erfindungsgemäßen Verfahrens (b) in einem größeren Bereich variiert werden. Im Allgemeinen arbeitet man bei Temperaturen zwischen -20°C und +150°C, vorzugsweise zwischen 0°C und +100°C.

Die Reaktionstemperaturen können bei der Durchführung des erfindungsgemäßen Verfahrens zur Herstellung der Zwischenprodukte der Formel (III) in einem größeren Bereich variiert werden. Im Allgemeinen arbeitet man bei Temperaturen zwischen 0°C und +150°C, vorzugsweise zwischen 10°C und +80°C.

Die erfindungsgemäßen Verfahren (a) und (b) und das Verfahren zur Herstellung Zwischenprodukte der Formel (III) werden im Allgemeinen unter Normaldruck durchgeführt. Es ist jedoch auch möglich, die erfindungsgemäßen Verfahren unter erhöhtem oder vermindertem Druck - im Allgemeinen zwischen 0,1 bar und 10 bar - durchzuführen.

Zur Durchführung der erfindungsgemäßen Verfahren (a) und (b) und des Verfahrens zur Herstellung Zwischenprodukte der Formel (III) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium. Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit anderen agrochemischen Wirkstoffen, z.B. bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flufenpyr, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Ketospiradox, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Penoxysulam, Pentoxazone, Pethoxamid, Phenmedipham, Picolinafen, Piperophos, Pretilachlor, Primisulfuron (-methyl), Profluazol, Profoxydim, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribehuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden, gegebenenfalls in Kombination mit konventionellen Methoden erhalten wurden (Genetically Modified Organisms) und deren Teile behandelt. Der Begriff "Teilen" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die durch konventionelle Züchtung, durch Mutagenese, oder auch durch rekombinante DNA-Techniken erhalten worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel - auch in Kombination mit anderen agrochemischen Wirkstoffen, besseres Wachstum der Kulturpflanzen, erhöhte Toleranz der Kulturpflanzen gegenüber hohen oder niedrigen Temperaturen, erhöhte Toleranz der Kulturpflanzen gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CrylA(a), CrylA(b), CrylA(c), CryllA, CrylllA, CrylllB2, Cry9c Cry2Ab, Cry3Bb und CrylF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinothricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinothricin, z.B. Raps), IMI® (Toleranz gegen Imidazolinone) und STS® (Toleranz gegen Sulfonylharnstoffe z.B. Mais) vertrieben werden. Als Herbizid-resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft mit den erfindungsgemäßen Verbindungen bzw. den erfindungsgemäßen Wirkstoffmischungen behandelt werden, wobei zusätzlich zu der guten Bekämpfung der Unkrautpflanzen die oben genannten synergistischen Effekte mit den transgenen Pflanzen oder Pflanzensorten auftreten. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den nachfolgenden Beispielen hervor.

### Herstellungsbeispiele:

### Beispiel 1

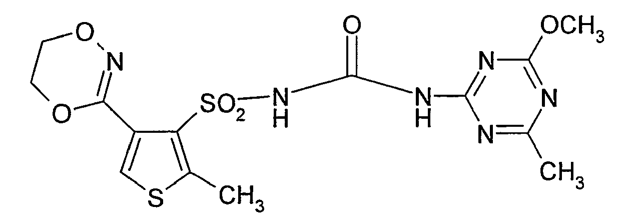

Zu einer Lösung von 0,41 g (1,59 mmol) 2-Phenoxycarbonylamino-4-methoxy-6-methyl-1,3,5-triazin in 40 ml Acetonitril werden bei Raumtemperatur (ca. 20 °C) nacheinander 0,50 g (1,91 mmol) 4-(5,6-dihydro-[1,4,2]-dioxazin-3-yl)-2-methyl-thienyl-3-sulfonamid und 0,29 g DBU (1,91 mmol) gegeben. Nach 12 Stunden Rühren bei Raumtemperatur wird das Lösungsmittel im Wasserstrahlvakuum entfernt, mit 100 ml Methylenchlorid versetzt, mit 2N Salzsäure und mit Wasser gewaschen, über Natriumsulfat getrocknet und schließlich das zugegebene Lösungsmittel im Wasserstrahlvakuum entfernt. Der Rückstand wird aus Isopropanol umkristallisiert, abfiltriert und getrocknet.

Man erhält 0,40 g (59% der Theorie) N-(4-Methoxy-6-methyl-1,3,5-triazin-2-yl)-N'-(4-(5,6-dihydro-[1,4,2]-dioxazin-3-yl)-2-methyl-thien-3-yl-sulfonyl)-harnstoff vom Schmelzpunkt 200 °C.

Analog zu Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) hergestellt werden.

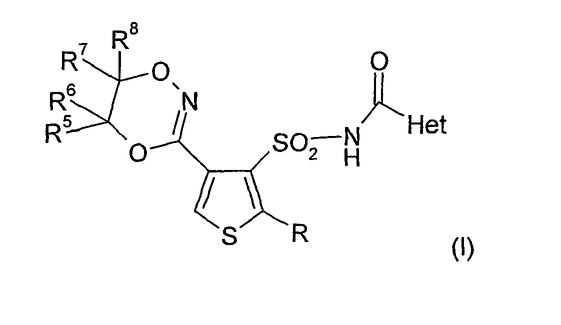

**Tabelle 1: Beispiele für die Verbindungen der Formel (I)**

| | | | | | | |
|---|---|---|---|---|---|---|
| Het = 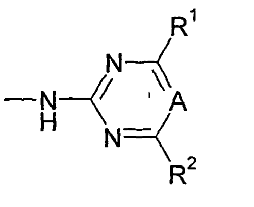 | | | | | | |

| Bsp.- Nr. | A | R¹ | R² | R | R⁵, R⁶, R⁷, R⁸ | Schmelzpunkt (°C) |
|---|---|---|---|---|---|---|
| 2 | CH | OCH₃ | OCH₃ | CH₃ | H | 211 |
| 3 | CH | OCH₃ | Cl | CH₃ | H | 97 |
| 4 | N | OCH₃ | OCH₃ | CH₃ | H | 199 |

### Beispiel 5

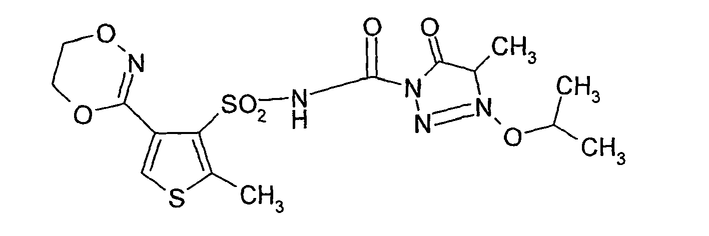

Zu einer Lösung von 0,44 g (1,59 mmol) 5-Isopropyloxy-4-methyl-2-phenoxy-carbonyl-2,4-dihydro-3H-1,2,4-triazol-3-on in 40 ml Acetonitril werden bei Raumtemperatur (ca. 20 °C) nacheinander 0,50 g (1,91 mmol) 4-(5,6-dihydro-[1,4,2]-dioxazin-3-yl)-2-methyl-thienyl-3-sulfonamid und 0,29 g DBU (1,91 mmol) gegeben. Nach 12 Stunden Rühren bei Raumtemperatur wird das Lösungsmittel im Wasserstrahlvakuum entfernt, mit 100 ml Methylenchlorid versetzt, mit 2N Salzsäure und mit Wasser gewaschen, über Natriumsulfat getrocknet und schließlich das zugegebene Lösungsmittel im Wasserstrahlvakuum entfernt. Der Rückstand wird aus Isopropanol umkristallisiert und abfiltriert und getrocknet.

Man erhält 0,5 g (71% der Theorie) 5-Isopropyloxy-4-methyl-2-[[4-(5,6-dihydro-[1,4,2]-dioxazin-3-yl)-2-methyl-thien-3-yl]-sulfonyl-amino-carbonyl]-2,4-dihydro-3H-1,2,4-triazol-3-on vom Schmelzpunkt 199 °C.

Analog zu Beispiel 5 sowie entsprechend der allgemeinen Beschreibung des erfindungsgemäßen Herstellungsverfahrens können beispielsweise auch die in der nachstehenden Tabelle 1 (Fortsetzung) aufgeführten Verbindungen der allgemeinen Formel (I) hergestellt werden.

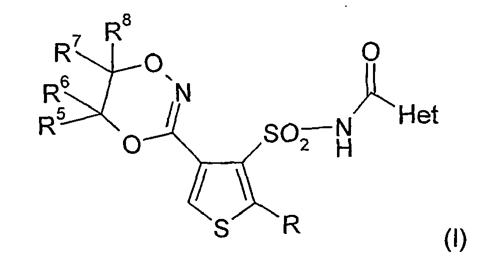

**Tabelle 1 (Fortsetzung): Beispiele für die Verbindungen der Formel (I)**

| | | | | | |
|---|---|---|---|---|---|
| Het = 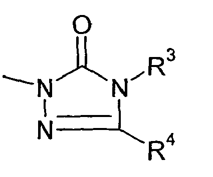 | | | | | |

| Bsp.- Nr. | R³ | R⁴ | R | R⁵, R⁶, R⁷, R⁸ | Schmelzpunkt (°C) |
|---|---|---|---|---|---|
| 6 | CH₃ | OCH₃ | CH₃ | H | 157 |
| 7 | CH₃ | OCH₂CH₃ | CH₃ | H | 185 |
| 8 | CH₃ | O-(n-Propyl) | CH₃ | H | 177 |
| 9 | 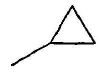 | OCH₃ | CH₃ | H | 204 |
| 10 | 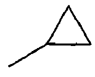 | OCH₂CH₃ | CH₃ | H | 202 |
| 11 | 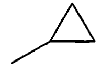 | O-(n-Propyl) | CH₃ | H | 190 |
| 12 | 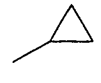 | O-(i-Propyl) | CH₃ | H | 200 |

### Ausgangsstoffe der Formel (III):

### Beispiel (lll-1)

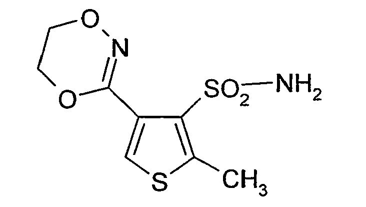

27,8 g (0,4 mol) Hydroxylamin-Hydrochlorid werden in 400 ml Methanol suspendiert und bei Raumtemperatur (ca. 20 °C) mit einer Lösung aus 44,8 g (0,8 mol) Kaliumhydroxid in 400 ml Methanol versetzt. Bei Raumtemperatur werden 47 g (0,7mol) 2-Methyl-3-sulfamoyl-thienyl-4-carbonsäuremethylester portionsweise hinzugegeben und für 12 Stunden bei 40 °C gerührt. Nach Abkühlen auf Raumtemperatur werden 27,6 g (0,2 mol) Kaliumcarbonat zugegeben und 185,8 g (0,92 mol) 1,2-Dibromethan zugetropft. Dann wird für weitere 12 Stunden bei 60° C gerührt. Nach Abkühlen auf Raumtemperatur wird das Lösungsmittel im Wasserstrahlvakuum abgezogen und der verbliebene Rückstand mit 400 ml Methylenchlorid und 200 ml einer 25%igen Natriumdihydrogenphosphat-Lösung digeriert. Der entstandene Niederschlag wird abfiltriert und getrocknet.

Man erhält 11,8 g (23% der Theorie) 4-(5,6-dihydro-[1,4,2]-dioxazin-3-yl)-2-methyl-thienyl-3-sulfonamid vom Schmelzpunkt 48 °C.

### Ausführungsbeispiele

### Beispiel A

### Post-emergence-Test

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) formulierten Testverbindungen werden in verschiedenen Dosierungen mit einer Wasseraufwandmenge von umgerechnet 800 I/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). In diesem Test zeigen beispielsweise die Verbindungen gemäß Herstellungsbeispiel 1, 3, 6, 7 und 9 sehr gute Wirkung gegen Unkräuter.

**Tabelle A1: Post emergence-Test/Gewächshaus**

| Wirkstoff gemäß Herstellungsbeispiel-Nr. | Aufwandmenge (g ai/ha) | Amaranthus | Lolium | Setaria | Sinapis | Stellaria | Cyperus | Echinochloa |
|---|---|---|---|---|---|---|---|---|
| (1) | 80 | 90 | 90 | 90 | 90 | 90 | 100 | 90 |
| (3) | 80 | 90 | 90 | 80 | 90 | 90 | 100 | 90 |
| (6) | 80 | 90 | 90 | 80 | 80 | 90 | 80 | 80 |
| (7) | 80 | 90 | 90 | 90 | 80 | 90 | 90 | 90 |
| (9) | 80 | 100 | 90 | 90 | 80 | 90 | 70 | 90 |

## Patentansprüche

1. Verbindungen der allgemeinen Formel (I) 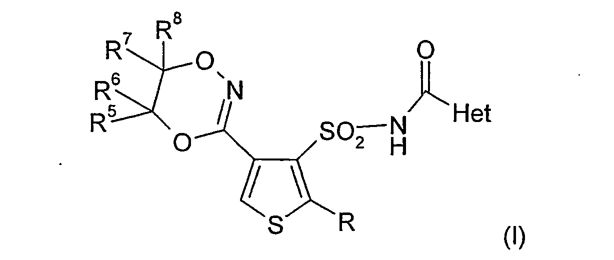 in welcher
Het für 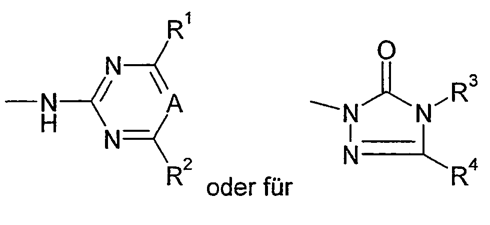 steht,
A für Stickstoff oder eine CH-Gruppierung steht,
R für Wasserstoff, Cyano, Nitro, Halogen, für jeweils gegebenenfalls durch Cyano, Halogen oder C₁-C₄-Alkoxy substituiertes Alkyl, Alkoxy, Alkoxycarbonyl, Alkylthio, Alkylsulfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
R¹ für Wasserstoff, für Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen; oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
R² für Wasserstoff, für Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
R³ für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Brom, Cyano, C₁-C₄-Alkoxy oder C₁-C₄-Alkoxy-carbonyl substituiertes Alkoxy, Alkylamino oder Alkyl-carbonylamino mit jeweils 1 bis 6 Kohlenstoffatomen in der Alkylgruppe, für Alkenyloxy mit 3 bis 6 Kohlenstoffatomen, für Dialkylamino mit jeweils 1 bis 4 Kohlenstoffatomen in den Alkylgruppen, für jeweils gegebenenfalls durch Fluor, Chlor, Brom, Cyano und/oder C₁-C₄-Alkyl substituiertes Cycloalkyl, Cycloalkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl und/oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht,
R⁴ für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Cyano, C₁-C₄-Alkoxy oder C₁-C₄-Alkoxycarbonyl substituiertes Alkoxy, Alkylthio, Alkylamino oder Alkylcarbonylamino mit jeweils 1 bis 6 Kohlenstoffatomen in der Alkylgruppe, für Alkenyloxy, Alkinyloxy, Alkenylthio, Alkinylthio, Alkenylamino oder Alkinylamino mit jeweils 3 bis 6 Kohlenstoffatomen in der Alkenyl- oder Alkinylgruppe, für Dialkylamino mit jeweils 1 bis 4 Kohlenstoffatomen in den Alkylgruppen, für jeweils gegebenenfalls durch Fluor, Chlor, Brom, Cyano und/oder C₁-C₄-Alkyl substituiertes Cycloalkyl, Cycloalkenyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in der Cycloalkyl- bzw. Cycloalkenylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil steht, und
R⁵, R⁶, R⁷ und R⁸ unabhängig voneinander für Wasserstoff, Halogen, Cyano oder Thiocyanato oder für gegebenenfalls durch Halogen substituiertes Alkyl, Alkoxy, Alkylthio, Alkylsulfinyl, Alkylsulfonyl Alkylamino, Alkylcarbonyl, Alkoxycarbonyl oder Alkylaminocarbonyl mit jeweils 1 bis 3 Kohlenstoffatomen im Alkylteil stehen,
sowie Salze von Verbindungen der Formel (I).

2. Verbindungen gemäß Anspruch 1, **dadurch gekennzeichnet, dass**
R für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, Methoxycarbonyl, Ethoxycarbonyl, n- oder i-Propoxy-carbonyl, , Methylthio, Ethylthio, n- oder i-Propylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy steht,
R¹ für Fluor, Chlor, Brom, Iod, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R² für Fluor, Chlor, Brom, Iod, oder für jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R³ für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht,
R⁴ für Wasserstoff, Hydroxy, Mercapto, Amino, Cyano, Fluor, Chlor, Brom, für jeweils gegebenenfalls durch Fluor, Chlor, Cyano, Methoxy, Ethoxy, n- oder i-Propoxy, Acetyl, Propionyl, n- oder i-Buyroyl, Methoxycarbonyl, Ethoxycarbonyl, n- oder i-Propoxy-carbonyl substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Ethinyl, Propinyl oder Butinyl, für jeweils gegebenenfalls durch Fluor, Chlor, Cyano, Methoxy, Ethoxy, n- oder i-Propoxy, Methoxycarbonyl, Ethoxycarbonyl, n- oder i-Propoxycarbonyl substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Acetylamino oder Propionylamino, für Propenyloxy, Butenyloxy, Ethinyloxy, Propinyloxy, Butinyloxy, Propenylthio, Butenylthio, Propinylthio, Butinylthio, Propenylamino, Butenylamino, Propinyl-amino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Brom, Methyl,'Trifluormethyl, Methoxy oder Methoxy-carbonyl substituiertes Phenyl, Benzyl, Phenoxy, Benzyloxy, Phenylthio, Benzylthio, Phenylamino oder Benzylamino steht, und
R⁵, R⁶, R⁷ und R⁸ stehen bevorzugt unabhängig voneinander für Wasserstoff oder Methyl stehen.
sowie die Natrium-, Kalium-, Magnesium-, Calcium-, Ammonium-, C₁-C₄-Alkylammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze dieser Verbindungen.

3. Verbindungen gemäß Anspruch 1, **dadurch gekennzeichnet, dass**
R für Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy steht,
R¹ für Fluor, Chlor, Brom, oder Iod, oder für gegebenenfalls durch Fluor oder Chlor substituiertes Methyl, Ethyl, n- oder i-Propyl, Methoxy, Ethoxy, n- oder i-Propoxy oder für Dimethylamino steht,
R² für Fluor, Chlor, Brom, oder Iod, oder für gegebenenfalls durch Fluor oder Chlor substituiertes Methyl, Ethyl, n- oder i-Propyl, Methoxy, Ethoxy, n- oder i-Propoxy oder für Dimethylamino steht,
R³ für Methyl, Ethyl, n- oder i-Propyl oder Cyclopropyl steht,
R⁴ für Methoxy, Ethoxy, n- oder i-Propoxy, n-, i-, s- oder t-Butoxy steht, und
R⁵, R⁶, R⁷ und R⁸ für Wasserstoff stehen,
sowie die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium, C₅- oder C₆-Cycloalkyl-ammonium- oder Di-(C₁-C₂-alkyl)-benzyl-ammoniumSalze von Verbindungen der Formel (1).

4. Verfahren zum Herstellen von Verbindungen gemäß Anspruch 1, **dadurch gekennzeichnet, dass**
(a) substituierte Aminoazine der allgemeinen Formel (II) 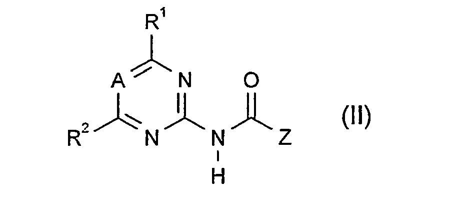 in welcher
A, R¹ und R² die in Anspruch 1 angegebene Bedeutung haben, und
Z für Halogen, Alkoxy oder Aryloxy steht,
mit Thiophenderivaten der allgemeinen Formel (III) 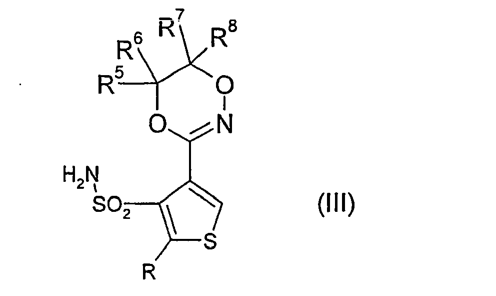 in welcher
R und R⁵ bis R⁸ die in Anspruch 1 angegebene Bedeutung haben,
gegebenenfalls in Gegenwart eines Reaktionshilfsmittels und gegebenenfalls in Gegenwart eines Verdünnungsmittels umgesetzt werden,
oder (b) substituierte Thiophen-3-sulfonamide der allgemeinen Formel (III) 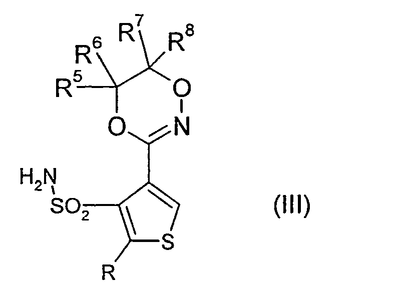 in welcher R und R⁵ bis R⁸ die in Anspruch 1 angegebene Bedeutung haben,
mit substituierten Triazolinonen der allgemeinen Formel (IV) 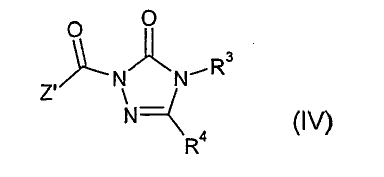 in welcher
R³ und R⁴ die in Anspruch 1 angegebene Bedeutung haben und
Z' für Halogen, Alkoxy, Aryloxy oder Arylalkoxy steht,
gegebenenfalls in Gegenwart eines Reaktionshilfsmittels und gegebenenfalls in Gegenwart eines Verdünnungsmittels umgesetzt werden,
und gegebenenfalls die nach den Verfahren (a) oder (b) erhaltenen Verbindungen der Formel (I) nach üblichen Methoden in Salze überführt werden.

5. Verfahren zum Bekämpfen von unerwünschtem Pflanzenwuchs, **dadurch gekennzeichnet, dass** man mindestens eine Verbindung gemäß einem der Ansprüche 1 bis 3 auf unerwünschte Pflanzen, Pflanzenteile und/oder ihren Lebensraum einwirken lässt.

6. Verwendung von mindestens einer Verbindung gemäß einem der Ansprüche 1 bis 3 zum Bekämpfen von unerwünschten Pflanzen.

7. Herbizides Mittel, **gekennzeichnet durch** einen Gehalt an einer oder mehreren Verbindungen gemäß einem der Ansprüche 1 bis 3 und üblichen Streckmitteln und/oder oberflächenaktiven Mitteln.

8. Herbizides Mittel, **gekennzeichnet durch** einen Gehalt an einer oder mehreren Verbindungen gemäß einem der Ansprüche 1 bis 3 und einem oder mehreren anderen agrochemischen Wirkstoffen.

9. Verbindungen der Formel (III) 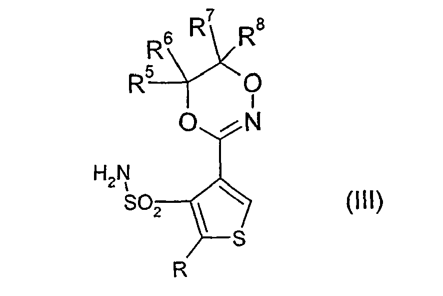 in welcher
R und R⁵ bis R⁸ die in Anspruch 1 angegebene Bedeutung haben.

10. Verwendung von Verbindungen der Formel (III) zur Herstellung von Verbindungen gemäß einem der Ansprüche 1 bis 3.

## Claims

1. A compound of the formula (I) 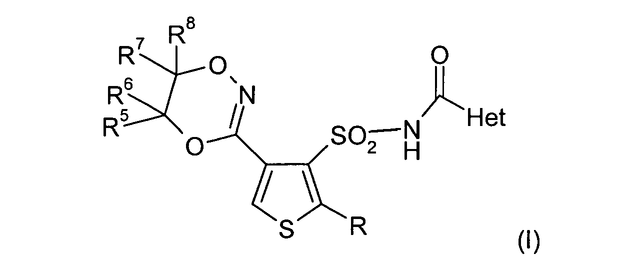 in which
Het is 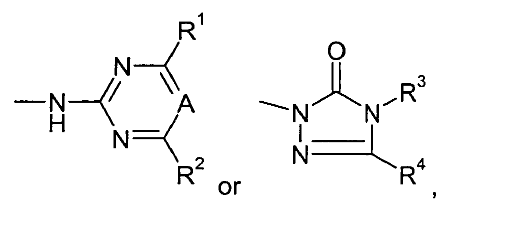
A is nitrogen or a CH grouping,
R is hydrogen, cyano, nitro, halogen, is in each case optionally cyano-, halogen- or C₁-C₄-alkoxy-substituted alkyl, alkoxy, alkoxycarbonyl, alkylthio, alkylsulfinyl or alkylsulfonyl having in each case 1 to 6 carbon atoms in the alkyl group, or is in each case optionally cyano- or halogen-substituted alkenyl, alkynyl, alkenyloxy or alkynyloxy having in each case 2 to 6 carbon atoms in the alkenyl or alkynyl group,
R¹ is hydrogen, is halogen, is in each case optionally cyano-, halogen- or C₁-C₄-alkoxy-substituted alkyl, alkoxy, alkylthio, alkylamino or dialkylamino having in each case 1 to 4 carbon atoms in the alkyl groups, or is in each case optionally cyano-, halogen-, C₁-C₄-alkyl- or C₁-C₄-alkoxy-substituted phenoxy, oxetanyloxy, furyloxy or tetrahydrofuryloxy,
R² is hydrogen, is halogen, is in each case optionally cyano-, halogen- or C₁-C₄-alkoxy-substituted alkyl, alkoxy, alkylthio, alkylamino or dialkylamino having in each case 1 to 4 carbon atoms in the alkyl groups, or is in each case optionally cyano-, halogen-, C₁-C₄-alkyl- or C₁-C₄-alkoxy-substituted phenoxy, oxetanyloxy, furyloxy or tetrahydrofuryloxy,
R³ is hydrogen, hydroxyl, amino, cyano, is C₂-C₁₀-alkylideneamino, is optionally fluorine-, chlorine-, bromine-, cyano-, C₁-C₄-alkoxy-, C₁-C₄-alkyl-carbonyl- or C₁-C₄-alkoxy-carbonyl-substituted alkyl having 1 to 6 carbon atoms, is in each case optionally fluorine-, chlorine- and/or bromine-substituted alkenyl or alkynyl having in each case 2 to 6 carbon atoms, is in each case optionally fluorine-, chlorine-, bromine-, cyano-, C₁-C₄-alkoxy- or C₁-C₄-alkoxy-carbonyl-substituted alkoxy, alkylamino or alkylcarbonylamino having in each case 1 to 6 carbon atoms in the alkyl group, is alkenyloxy having 3 to 6 carbon atoms, is dialkylamino having in each case 1 to 4 carbon atoms in the alkyl groups, is in each case optionally fluorine-, chlorine-, bromine-, cyano- and/or C₁-C₄-alkyl-substituted cycloalkyl, cycloalkylamino or cycloalkylalkyl having in each case 3 to 6 carbon atoms in the alkyl group and, if appropriate, 1 to 4 carbon atoms in the alkyl moiety, or is in each case optionally fluorine-, chlorine-, bromine-, cyano-, nitro-, C₁-C₄-alkyl-, trifluoromethyl- and/or C₁-C₄-alkoxy-substituted aryl or arylalkyl having in each case 6 or 10 carbon atoms in the aryl group and, if appropriate, 1 to 4 carbon atoms in the alkyl moiety.
R⁴ is hydrogen, hydroxyl, mercapto, amino, cyano, fluorine, chlorine, bromine, iodine, is optionally fluorine-, chlorine-, bromine-, cyano-, C₁-C₄-alkoxy-, C₁-C₄-alkyl-carbonyl- or C₁-C₄-alkoxy-carbonyl-substituted alkyl having 1 to 6 carbon atoms, is in each case optionally fluorine-, chlorine- and/or bromine-substituted alkenyl or alkynyl having in each case 2 to 6 carbon atoms, is in each case optionally fluorine-, chlorine-, cyano-, C₁-C₄-alkoxy- or C₁-C₄-alkoxy-carbonyl-substituted alkoxy, alkylthio, alkylamino or alkylcarbonylamino having in each case 1 to 6 carbon atoms in the alkyl group, is alkenyloxy, alkynyloxy, alkenylthio, alkynylthio, alkenylamino or alkynylamino having in each case 3 to 6 carbon atoms in the alkenyl or alkynyl group, is dialkylamino having in each case 1 to 4 carbon atoms in the alkyl groups, is in each case optionally fluorine-, chlorine-, bromine-, cyano- and/or C₁-C₄-alkyl-substituted cycloalkyl, cycloalkenyl, cycloalkyloxy, cycloalkylthio, cycloalkylamino, cycloalkylalkyl, cycloalkylalkoxy, cycloalkylalkylthio or cycloalkylalkylamino having in each case 3 to 6 carbon atoms in the cycloalkyl or cycloalkenyl group and, if appropriate, 1 to 4 carbon atoms in the alkyl moiety, or is in each case optionally fluorine-, chlorine-, bromine-, cyano-, nitro-, C₁-C₄-alkyl-, trifluoromethyl-, C₁-C₄-alkoxy- and/or C₁-C₄-alkoxy-carbonyl-substituted aryl, arylalkyl, aryloxy, arylalkoxy, arylthio, arylalkylthio, arylamino or arylalkylamino having in each case 6 or 10 carbon atoms in the aryl group and, if appropriate, 1 to 4 carbon atoms in the alkyl moiety, and
R⁵, R⁶, R⁷ and R⁸ independently of one another are hydrogen, halogen, cyano or thiocyanato or are optionally halogen-substituted alkyl, alkoxy, alkylthio, alkylsulfinyl, alkylsulfonyl, alkylamino, alkylcarbonyl, alkoxycarbonyl or alkylaminocarbonyl having in each case 1 to 3 carbon atoms in the alkyl moiety,
or a salt of a compound of the formula (1).

2. The compound as claimed in claim 1, wherein
R is hydrogen, cyano, fluorine, chlorine, bromine, is in each case optionally cyano-, fluorine-, chlorine-, methoxy- or ethoxy-substituted methyl, ethyl, n- or i-propyl, n-, i-, s- or t-butyl, methoxy, ethoxy, n- or i-propoxy, methoxycarbonyl, ethoxycarbonyl, n- or i-propoxycarbonyl, methylthio, ethylthio, n- or i-propylthio, methylsulfinyl, ethylsulfinyl, methylsulfonyl or ethylsulfonyl, or is in each case optionally cyano-, fluorine- or chlorine-substituted propenyl, butenyl, propynyl, butynyl, propenyloxy, butenyloxy, propynyloxy or butynyloxy,
R¹ is fluorine, chlorine, bromine, iodine, or is in each case optionally cyano-, fluorine-, chlorine-, methoxy- or ethoxy-substituted methyl, ethyl, n- or i-propyl, methoxy, ethoxy, n- or i-propoxy, methylthio, ethylthio, n- or i-propylthio, methylamino, ethylamino, n- or i-propylamino, dimethylamino or diethylamino,
R² is fluorine, chlorine, bromine, iodine, or is in each case optionally cyano-, fluorine-, chlorine-, methoxy- or ethoxy-substituted methyl, ethyl, n- or i-propyl, methoxy, ethoxy, n- or i-propoxy, methylthio, ethylthio, n- or i-propylthio, methylamino, ethylamino, n- or i-propylamino, dimethylamino or diethylamino,
R³ is hydrogen, hydroxyl, amino, is in each case optionally fluorine-, chlorine-, cyano-, methoxy- or ethoxy-substituted methyl, ethyl, n- or i-propyl, n-, i-, s- or t-butyl, is in each case optionally fluorine-, chlorine- and/or bromine-substituted ethenyl, propenyl, butenyl, propynyl or butynyl, is in each case optionally fluorine-, chlorine-, cyano-, methoxy- or ethoxy-substituted methoxy, ethoxy, n- or i-propoxy, n-, i-, s- or t-butoxy, methylamino, ethylamino, n- or i-propylamino, n-, i-, s- or t-butylamino, is propenyloxy or butenyloxy, is dimethylamino or diethylamino, is in each case optionally fluorine-, chlorine-, methyl- and/or ethyl-substituted cyclopropyl, cyclobutyl, cyclopentyl, cyclohexyl, cyclopropylamino, cyclobutylamino, cyclopentylamino, cyclohexylamino, cyclopropylmethyl, cyclobutylmethyl, cyclopentylmethyl or cyclohexylmethyl, or is in each case optionally fluorine-, chlorine-, methyl-, trifluoromethyl- and/or methoxy-substituted phenyl or benzyl,
R⁴ is hydrogen, hydroxyl, mercapto, amino, cyano, fluorine, chlorine, bromine, is in each case optionally fluorine-, chlorine-, cyano-, methoxy-, ethoxy-, n- or i-propoxy-, acetyl-, propionyl-, n- or i-butyroyl-, methoxycarbonyl-, ethoxycarbonyl-, n- or i-propoxycarbonyl-substituted methyl, ethyl, n- or i-propyl, n-, i-, s- or t-butyl, is in each case optionally fluorine-, chlorine- and/or bromine-substituted ethenyl, propenyl, butenyl, ethynyl, propynyl or butynyl, is in each case optionally fluorine-, chlorine-, cyano-, methoxy-, ethoxy-, n- or i-propoxy-, methoxycarbonyl-, ethoxycarbonyl-, n- or i-propoxycarbonyl-substituted methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, acetylamino or propionylamino, is propenyloxy, butenyloxy, ethynyloxy, propynyloxy, butynyloxy, propenylthio, butenylthio, propynylthio, butynylthio, propenylamino, butenylamino, propynylamino or butynylamino, is dimethylamino, diethylamino or dipropylamino, is in each case optionally fluorine-, chlorine-, methyl- and/or ethyl-substituted cyclopropyl, cyclobutyl, cyclopentyl, cyclohexyl, cyclopentenyl, cyclohexenyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentyl-methyl, cyclohexylmethyl, cyclopropylmethoxy, cyclobutylmethoxy, cyclopentylmethoxy, cyclohexylmethoxy, cyclopropylmethylthio, cyclo-butylmethylthio, cyclopentylmethylthio, cyclohexylmethylthio, cyclo-propylmethylamino, cyclobutylmethylamino, cyclopentylmethylamino or cyclohexylmethylamino, or is in each case optionally fluorine-, chlorine-, bromine-, methyl-, trifluoromethyl-, methoxy- or methoxy-carbonyl-substituted phenyl, benzyl, phenoxy, benzyloxy, phenylthio, benzylthio, phenylamino or benzylamino, and
R⁵, R⁶, R⁷ and R⁸ independently of one another are hydrogen or methyl,
or a sodium, potassium, magnesium, calcium, ammonium-, C₁-C₄-alkylammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulfonium, C₅- or C₆-cycloalkyl-ammonium or di-(C₁-C₂-alkyl)-benzylammonium salt of this compound.

3. The compound as claimed in claim 1, wherein
R is fluorine, chlorine, bromine, is in each case optionally cyano-, fluorine-, chlorine-, methoxy- or ethoxy-substituted methyl, ethyl, n- or i-propyl, n-, i-, s- or t-butyl, methoxy, ethoxy, n- or i-propoxy,
R¹ is fluorine, chlorine, bromine, or iodine, or is optionally fluorine- or chlorine-substituted methyl, ethyl, n- or i-propyl, methoxy, ethoxy, n- or i-propoxy or is dimethylamino,
R² is fluorine, chlorine, bromine, or iodine, or is optionally fluorine- or chlorine-substituted methyl, ethyl, n- or i-propyl, methoxy, ethoxy, n- or i-propoxy or is dimethylamino,
R³ is methyl, ethyl, n- or i-propyl or cyclopropyl,
R⁴ is methoxy, ethoxy, n- or i-propoxy, n-, i-, s- or t-butoxy, and
R⁵, R⁶, R⁷ and R⁸ are hydrogen,
or a sodium, potassium, magnesium, calcium, ammonium-, C₁-C₄-alkylammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulfonium, C₅- or C₆-cycloalkyl-ammonium or di-(C₁-C₂-alkyl)-benzylammonium salt of a compound of the formula (I).

4. A process for preparing compounds as claimed in claim 1, which comprises
(a) reacting substituted aminoazines of the formula (II) 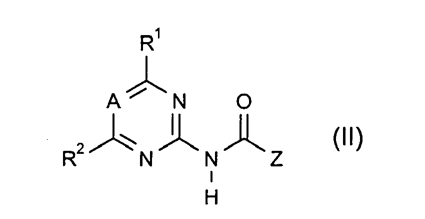 in which
A, R¹ and R² are
as defined in claim 1 and
Z is halogen, alkoxy or aryloxy
with thiophene derivatives of the formula (III) 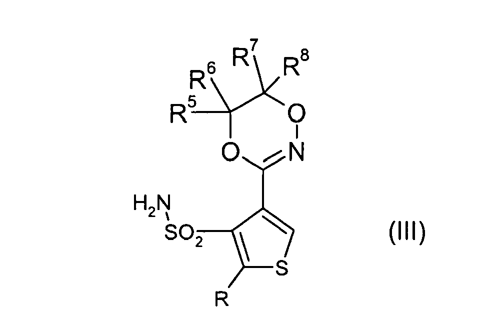 in which
R and R⁵ to R⁸ are as defined in claim 1,
if appropriate in the presence of a reaction auxiliary and if appropriate in the presence of a diluent,
or (b) reacting substituted thiophene-3-sulfonamides of the formula (III) 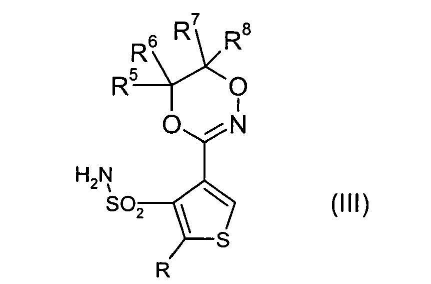 in which R and R⁵ to R⁸ are as defined in claim 1
with substituted triazolinones of the formula (IV) 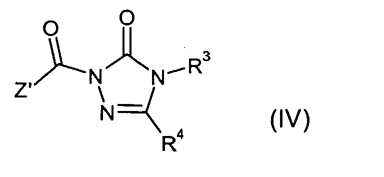 in which
R³ and R⁴ are as defined in claim 1 and
Z' is halogen, alkoxy, aryloxy or arylalkoxy,
if appropriate in the presence of a reaction auxiliary and if appropriate in the presence of a diluent,
and, if appropriate, converting the compounds of the formula (I) obtained by process (a) or (b) by customary methods into salts.

5. A method for controlling unwanted vegetation, which comprises allowing at least one compound as claimed in any of claims 1 to 3 to act on unwanted plants, parts of plants and/or their habitat.

6. The use of at least one compound as claimed in any of claims 1 to 3 for controlling unwanted plants.

7. A herbicidal composition, which comprises one or more compounds as claimed in any of claims 1 to 3 and customary extenders and/or surfactants.

8. A herbicidal composition, which comprises one or more compounds as claimed in any of claims 1 to 3 and one or more other agrochemically active compounds.

9. A compound of the formula (III) 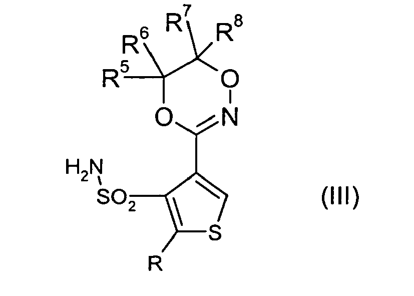 in which
R and R⁵ to R⁸ are as defined in claim 1.

10. The use of compounds of the formula (III) for preparing compounds as claimed in any of claims 1 to 3.

## Revendications

1. Composés de formule générale (I) 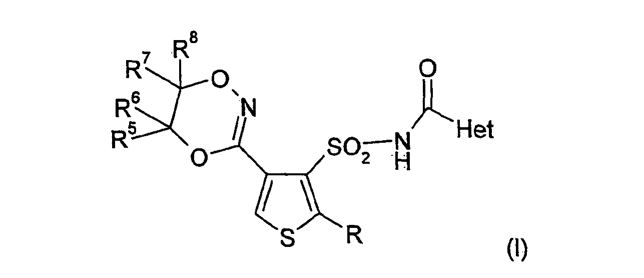 dans laquelle
Het désigne 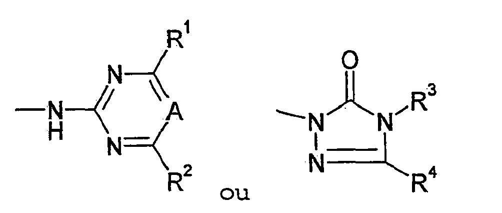 A un atome d'azote ou un groupe CH,
R désigne un atome d'hydrogène, un groupe cyano, nitro, un atome d'halogène, respectivement un groupe alkyle, alcoxy, alcoxycarbonyle, alkylthio, alkylsulfinyle ou alkylsulfonyle éventuellement substitué par un groupe cyano, halogène ou alcoxy en C₁ à C₄, comportant respectivement 1 à 6 atomes de carbone dans le groupe alkyle, ou respectivement un groupe alcényle, alcinyle, alcényloxy ou alcinyloxy éventuellement substitué par un groupe cyano ou halogène, comportant respectivement 2 à 6 atomes de carbone dans le groupe alcényle ou alcinyle,
R¹ désigne un atome d'hydrogène, d'halogène, respectivement un groupe alkyle, alcoxy, alkylthio, alkylamino ou dialkylamino éventuellement substitué par un groupe cyano, un atome d'halogène ou un groupe alcoxy en C₁ à C₄, comportant respectivement 1 à 4 atomes de carbone dans les groupes alkyle, ou respectivement un groupe phénoxy, oxétanyloxy, furyloxy ou tétrahydrofuryloxy éventuellement substitué par un groupe cyano, halogène, alkyle en C₁ à C₄ ou alcoxy en C₁ à C₄,
R² désigne un atome d'hydrogène, d'halogène, respectivement un groupe alkyle, alcoxy, alkylthio, alkylamino ou dialkylamino éventuellement substitué par un groupe cyano, un atome d'halogène ou un groupe alcoxy en C₁ à C₄, comportant respectivement 1 à 4 atomes de carbone dans les groupes alkyle, ou respectivement un groupe phénoxy, oxétanyloxy, furyloxy ou tétrahydrofuryloxy éventuellement substitué par un groupe cyano, un atome d'halogène, un groupe alkyle en C₁ à C₄ ou alcoxy en C₁ à C₄,
R³ désigne un atome d'hydrogène, un groupe hydroxy, amino, cyano, un groupe alkylidène-amino en C₂ à C₁₀, un groupe alkyle éventuellement substitué par un atome de fluor, de chlore, de brome, un groupe cyano, alcoxy en C₁ à C₄, alkyl-carbonyle en C₁ à C₄ ou alcoxycarbonyle en C₁ à C₄, comportant 1 à 6 atomes de carbone, respectivement un groupe alcényle ou alcinyle éventuellement substitué par un atome de fluor, de chlore et/ou de brome, comportant respectivement 2 à 6 atomes de carbone, un groupe alcoxy, alkylamino ou alkyl-carbonylamino éventuellement substitué par un atome de fluor, de chlore, de brome, un groupe cyano, alcoxy en C₁ à C₄ ou alcoxy carbonyle en C₁ à C₄, comportant respectivement 1 à 6 atomes de carbone dans le groupe alkyle, un groupe alcényloxy comportant 3 à 6 atomes de carbone, un groupe dialkylamino comportant respectivement 1 à 4 atomes de carbone dans les groupes alkyle, respectivement un groupe cycloalkyle, cycloalkylamino ou cycloalkylalkyle éventuellement substitué par un atome de fluor, de chlore, de brome, un groupe cyano et/ou alkyle en C₁ à C₄, comportant respectivement 3 à 6 atomes de carbone dans le groupe alkyle et éventuellement 1 à 4 atomes de carbone dans la partie alkyle, ou un groupe aryle ou arylalkyle éventuellement substitué par un atome de fluor, de chlore, de brome, un groupe cyano, nitro, alkyle en C₁ à C₄, trifluorométhyle et/ou alcoxy en C₁ à C₄, comportant respectivement 6 ou 10 atomes de carbone dans le groupe aryle et éventuellement, 1 à 4 atomes de carbone dans la partie alkyle,
R⁴ désigne un atome d'hydrogène, un groupe hydroxy, mercapto, amino, cyano, un atome de fluor, de chlore, de brome, d'iode, un groupe alkyle éventuellement substitué par un atome de fluor, de chlore, de brome, un groupe cyano, alcoxy en C₁ à C₄, alkyl-carbonyle en C₁ à C₄ ou alcoxy-carbonyle en C₁ à C₄ comportant 1 à 6 atomes de carbone, respectivement un groupe alcényle ou alcinyle éventuellement substitué par un atome de fluor, de chlore et/ou de brome, comportant respectivement 2 à 6 atomes de carbone, respectivement un groupe alcoxy, alkylthio, alkylamino ou alkylcarbonylamino éventuellement substitué par un atome de fluor, de chlore, un groupe cyano, alcoxy en C₁ à C₄ ou alcoxycarbonyle en C₁ à C₄ comportant respectivement 1 à 6 atomes de carbone dans le groupe alkyle, un groupe alcényloxy, alcinyloxy, alcénylthio, alcinylthio, acénylamino ou alcinylamino comportant respectivement 3 à 6 atomes de carbone dans le groupe alcényle ou alcinyle, un groupe dialkylamino comportant respectivement 1 à 4 atomes de carbone dans les groupes alkyle, respectivement un groupe cycloalkyle, cycloalcényle, cycloalkyloxy, cycloalkylthio, cycloalkylamino, cycloalkylalkyle, cycloalkylalcoxy, cycloalkylalkylthio ou cycloalkylalkylamino éventuellement substitué par un atome de fluor, de chlore, de brome, un groupe cyano et/ou alkyle en C₁ à C₄, comportant respectivement 3 à 6 atomes de carbone dans le groupe cycloalkyle ou cycloalcényle et éventuellement 1 à 4 atomes de carbone dans la partie alkyle, ou respectivement un groupe aryle, arylalkyle, aryloxy, arylalcoxy, arylthio, arylalkylthio, arylamino ou arylalkylamino éventuellement substitué par un atome de fluor, de chlore, de brome, un groupe cyano, nitro, alkyle en C₁ à C₄, trifluorométhyle, alcoxy en C₁ à C₄ et/ou alcoxycarbonyle en C₁ à C₄, comportant respectivement 6 ou 10 atomes de carbone dans le groupe aryle et éventuellement 1 à 4 atomes de carbone dans la partie alkyle, et
R⁵, R⁶, R⁷ et R⁸, indépendamment les uns des autres, désignent un atome d'hydrogène, d'halogène, un groupe cyano ou thiocyanato ou un groupe alkyle, alcoxy, alkylthio, alkylsulfinyle, alkylsulfonyle, alkylamino, alkylcarbonyle, alcoxycarbonyle ou alkylaminocarbonyle éventuellement substitué par un atome d'halogène, comportant respectivement 1 à 3 atomes de carbone dans la partie alkyle,
et les sels des composés de formule (I).

2. Composés selon la revendication 1, **caractérisés en ce que**
R désigne un atome d'hydrogène, un groupe cyano, un atome de fluor, de chlore, de brome, un groupe méthyle, éthyle, n- ou i-propyle, n-, i-, s- ou t-butyle, méthoxy, éthoxy, n- ou i-propoxy, méthoxycarbonyle, éthoxycarbonyle, n- ou i-propoxycarbonyle, méthylthio, éthylthio, n- ou i-propylthio, méthylsulfinyle, éthyl-sulfinyle, méthylsulfonyle ou éthylsulfonyle éventuellement substitué par un groupe cyano, un atome de fluor, de chlore, un groupe méthoxy ou éthoxy, ou respectivement un groupe propényle, butényle, propinyle, butinyle, propényloxy, butényloxy, propinyloxy ou butinyloxy éventuellement substitué par un groupe cyano, un atome de fluor ou de chlore,
R¹ désigne un atome de fluor, de chlore, de brome, d'iode ou un groupe méthyle, éthyle, n- ou i-propyle, méthoxy, éthoxy, n- ou i-propoxy, méthylthio, éthylthio, n- ou i-propylthio, méthylamino, éthylamino, n- ou i-propylamino, diméthylamino ou diéthylamino éventuellement substitué par un groupe cyano, un atome de fluor, de chlore, un groupe méthoxy ou éthoxy,
R² désigne un atome de fluor, de chlore, de brome, d'iode ou un groupe méthyle, éthyle, n- ou i-propyle, méthoxy, éthoxy, n- ou i-propoxy, méthylthio, éthylthio, n- ou i-propylthio, méthylamino, éthylamino, n- ou i-propylamino, diméthylamino ou diéthylamino éventuellement substitué par un groupe cyano, un atome de fluor, de chlore, un groupe méthoxy ou éthoxy,
R³ désigne un atome d'hydrogène, un groupe hydroxy, amino, un groupe méthyle, éthyle, n- ou i-propyle, n-, i-, s- ou t-butyle éventuellement substitué par un atome de fluor, de chlore, un groupe cyano, méthoxy ou éthoxy ; un groupe éthényle, propényle, butényle, propinyle ou butinyle éventuellement substitué par un atome de fluor, de chlore et/ou de brome ; un groupe méthoxy, éthoxy, n- ou i-propoxy, n-, i-, s- ou t-butoxy, méthylamino, éthylamino, n- ou i-propylamino, n-, i-, s- ou t-butylamino éventuellement substitué par un atome de fluor, de chlore, un groupe cyano, méthoxy ou éthoxy ; un groupe propényloxy ou butényloxy ; un groupe diméthylamino ou diéthylamino ; respectivement un groupe cyclopropyle, cyclobutyle, cyclopentyle, cyclohexyle, cyclopropylamino, cyclobutylamino, cyclopentylamino, cyclohexylamino, cyclopropylméthyle, cyclobutylméthyle, cyclopebtylméthyle ou cyclohexylméthyle éventuellement substitué par un atome de fluor, de chlore, un groupe méthyle et/ou éthyle, ou respectivement un groupe phényle ou benzyle éventuellement substitué par un atome de fluor, de chlore, un groupe méthyle, trifluorométhyle et/ou méthoxy,
R⁴ désigne un atome d'hydrogène, un groupe hydroxy, mercapto, amino, cyano, un atome de fluor, de chlore, de brome, respectivement un groupe méthyle, éthyle, n- ou i-propyle, n-, i-, s- ou t-butyle éventuellement substitué par un atome de fluor, de chlore, un groupe cyano, méthoxy, éthoxy, n- ou i-propoxy, acétyle, propionyle, n- ou i-butyroyle, méthoxycarbonyle, éthoxycarbonyle, n- ou i-propoxy-carbonyle, respectivement un groupe éthényle, propényle, butényle, éthinyle, propinyle ou butinyle éventuellement substitué par un atome de fluor, de chlore et/ou de brome, respectivement un groupe méthoxy, éthoxy, n- ou i-propoxy, n-, i-, s- ou t-butoxy, méthylthio, éthylthio, n- ou i-propylthio, n-, i-, s- ou t-butylthio, méthylamino, éthylamino, n- ou i-propylamino, n-, i-, sou t-butylamino, acétylamino ou propionylamino éventuellement substitué par un atome de fluor, de chlore, un groupe cyano, méthoxy, éthoxy, n- ou i-propoxy, méthoxycarbonyle, éthoxycarbonyle, n- ou i-propoxycarbonyle ; un groupe propényloxy, butényloxy, éthinyloxy, propinyloxy, butinyloxy, propénylthio, buténylthio, propinylthio, butinylthio, propénylamino, buténylamino, propinylamino ou butinylamino ; un groupe diméthylamino, diéthylamino ou dipropylamino, respectivement un groupe cyclopropyle, cyclobutyle, cyclopentyle, cyclohexyle, cyclopentényle, cyclohenéxyle, cyclopropyloxy, cyclobutyloxy, cyclopentylaoxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentyl-méthoxy, cyclohexylméthoxy, cyclopropylméthylthio, cyclo-butylméthylthio, cyclopentylméthylthio, cyclohexylméthylthio, cyclopropylméthylamino, cyclobutylméthylamino, cyclopentylméthylamino ou cyclohexylméthylamino éventuellement substitué par un atome de fluor, de chlore, un groupe méthyle et/ou éthyle, ou respectivement un groupe phényle, benzyle, phénoxy, benzyloxy, phénylthio, benzylthio, phénylamino ou benzylamino éventuellement substitué par un atome de fluor, de chlore, de brome, un groupe méthyle, trifluorométhyle, méthoxy ou méthoxycarbonyle, et
R⁵, R⁶ R⁷ et R⁸ désignent de préférence indépendamment les uns des autres, un atome d'hydrogène ou un groupe méthyle,
et les sels de sodium, potassium, magnésium, calcium, ammonium, alkylammonium en C₁ à C₄, di-(alkyle en C₁ à C₄)-ammonium, tri-(alkyle en C₁ à C₄)-ammonium, tétra-(alkyle en C₁ à C₄)-ammonium, tri-(alkyle en C₁ à C₄)-sulfonium, cycloalkyl-ammonium en C₅ ou C₆ et di-(alkyle en C₁ à C₂)-benzyl-ammonium de ces composés.

3. Composés selon la revendication 1, **caractérisés en ce que**
R désigne un atome de fluor, de chlore, de brome, respectivement un groupe méthyle, éthyle, n- ou i-propyle, n-, i-, s- ou t-butyle, méthoxy, éthoxy, n- ou i-propoxy éventuellement substitué par un groupe cyano, un atome de fluor, de chlore, un groupe méthoxy ou éthoxy,
R¹ désigne un atome de fluor, de chlore, de brome ou d'iode ou un groupe méthyle, éthyle, n- ou i-propyle, méthoxy, éthoxy, n- ou i-propoxy éventuellement substitué par un atome de fluor ou de chlore, ou un groupe diméthylamino,
R² désigne un atome de fluor, de chlore, de brome ou d'iode ou un groupe méthyle, éthyle, n- ou i-propyle, méthoxy, éthoxy, n- ou i-propoxy éventuellement substitué par un atome de fluor ou de chlore, ou un groupe diméthylamino,
R³ désigne un groupe méthyle, éthyle, n- ou i-propyle ou cyclopropyle,
R⁴ désigne un groupe méthoxy, éthoxy, n- ou i-propoxy, n-, i-, s- ou t-butoxy, et
R⁵, R⁶, R⁷ et R⁸ désignent un atome d'hydrogène,
et les sels de sodium, potassium, magnésium, calcium, ammonium, alkylammonium en C₁ à C₄, di-(alkyle en C₁ à C₄) -ammonium, tri- (alkyle en C₁ à C₄) -ammonium, tétra-(alkyle en C₁ à C₄)-ammonium, tri-(alkyle en C₁ à C₄)-sulfonium, cycloalkyl-ammonium en C₅ ou C₆ et di-(alkyle en C₁ à C₂)-benzyl-ammonium des composés de formule (I).

4. Procédé de production de composés selon la revendication 1, **caractérisé en ce que**
(a) des amino-azines substituées de formule générale (II) 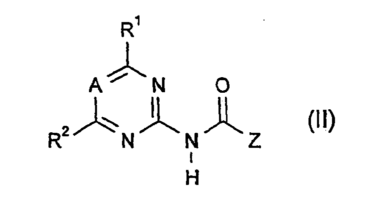 dans laquelle
A, R¹ et R² ont la signification indiquée à la revendication 1, et
Z désigne un atome d'halogène, un groupe alcoxy ou aryloxy,
sont converties avec des dérivés de thiophène de formule générale (III) 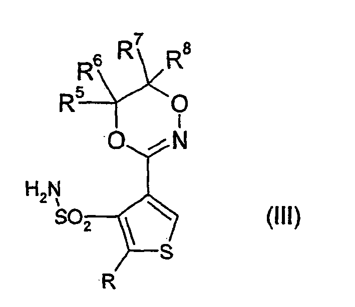 dans laquelle
R et R⁵ à R⁸ ont la signification mentionnée à la revendication 1,
éventuellement en présence d'un auxiliaire de réaction et éventuellement en présence d'un diluant,
ou (b) des thiophène-3-sulfonamides substitués de formule générale (III) 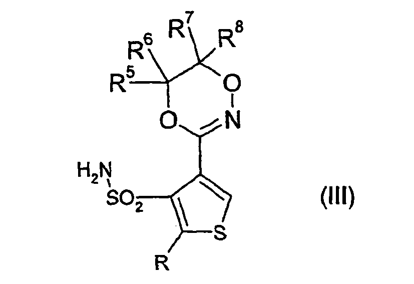 dans laquelle R et R⁵ à R⁸ ont la signification mentionnée à la revendication 1,
sont convertis avec des triazolinones substituées de formule générale (IV) 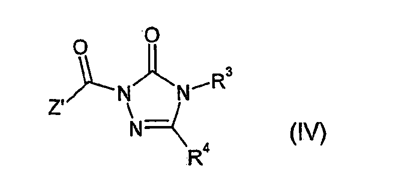 dans laquelle
R³ et R⁴ ont la signification mentionnée à la revendication 1, et
Z' désigne un atome d'halogène, un groupe alcoxy, aryloxy ou arylalcoxy,
éventuellement en présence d'un auxiliaire de réaction et éventuellement en présence d'un diluant,
et éventuellement, les composés obtenus selon le procédé (a) ou (b) sont convertis en sels selon les procédés habituels.

5. Procédé de lutte contre la croissance de plantes indésirable, **caractérisé en ce que** l'on laisse agir au moins un composé selon l'une des revendications 1 à 3 sur les plantes indésirables, les parties de plante et/ou leur milieu de vie.

6. Utilisation d'au moins un composé selon l'une quelconque des revendications 1 à 3, pour lutter contre les plantes indésirables.

7. Agent herbicide, **caractérisé par** une teneur en un ou plusieurs composés selon l'une quelconque des revendications 1 à 3, et agent de charge et/ou agent tensioactif habituel.

8. Agent herbicide **caractérisé par** une teneur en un ou plusieurs composés selon l'une quelconque des revendications 1 à 3, et un ou plusieurs autres principes actifs agrochimiques.

9. Composé de formule (III) 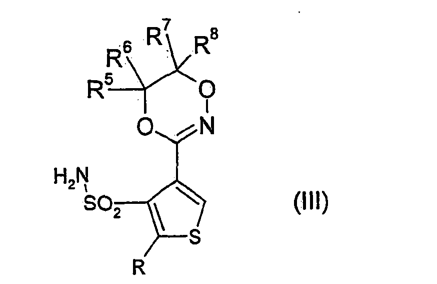 dans laquelle
R et R⁵ à R⁸ ont la signification mentionnée à la revendication 1.

10. Utilisation des composés de formule (III) pour la production de composés selon l'une quelconque des revendications 1 à 3.
